(19)

**Europäisches Patentamt**

**European Patent Office**

**Office européen des brevets**

(11) **EP 1 134 701 A2**

(12) **EUROPEAN PATENT APPLICATION**

(43) Date of publication:
**19.09.2001 Bulletin 2001/38**

(51) Int Cl.⁷: $G06T\ 17/00$

(21) Application number: **01302391.6**

(22) Date of filing: **15.03.2001**

| (84) Designated Contracting States: | (72) Inventors: |
|---|---|

(84) Designated Contracting States:
**AT BE CH CY DE DK ES FI FR GB GR IE IT LI LU MC NL PT SE TR**
Designated Extension States:
**AL LT LV MK RO SI**

(30) Priority: **15.03.2000 JP 2000072088**

(71) Applicant: **Infiniteface Inc.**
**New York, NY 10003 (US)**

(72) Inventors:
• **Furuta, Hima**
  **Minato-ku, Tokyo 106-0032 (JP)**
• **Miyazawa, Takeo**
  **Mitaka-shi, Tokyo 181-0013 (JP)**

(74) Representative: **Brown, Kenneth Richard et al**
**R.G.C. Jenkins & Co.**
**26 Caxton Street**
**London SW1H 0RJ (GB)**

(54) **Client-server system for three-dimensional face make-up simulation**

(57)     It is an object of the invention to provide a three-dimensional beauty simulation client-server system which is capable of handling a user's face in a three-dimensional fashion and of providing more realistic beauty simulations. This system comprises a shop-based client that obtains and transmits three-dimensional shape data regarding a user, and a server comprising a makeup simulation unit that receives and stores the three-dimensional shape data from the shop-based client and carries out makeup simulation based on the three-dimensional shape data in response to requests from the user, and a data control unit that analyzes the user's operation record and generates administrative information.

F I G. 1

**Description**

BACKGROUND OF THE INVENTION

1. Field of the Invention

The present invention relates to a three-dimensional beauty simulation client-server system to carry out beauty simulations based on a user's face model data.

2. Description of the Related Art

The invention described in Japanese Patent Laid-Open No. H6-319613, for example, comprises a conventional beauty simulation apparatus. This invention discloses a face makeup support apparatus using which makeup may be applied to a displayed face by simulating lipstick application, face powdering and eyebrow shaping on the image of a face displayed in an image display apparatus.

The conventional beauty simulation apparatus entails the problem that it can only carry out flat image processing, and does not appear realistic. Furthermore, it cannot be used over a computer network.

SUMMARY OF THE INVENTION

The present invention was created in order to resolve these problems, and an object thereof is to provide a three-dimensional beauty simulation client-server system that can display a user's face in a three-dimensional fashion and provide a more realistic beauty simulation.

The three-dimensional beauty simulation client-server system pertaining to the present invention includes a shop-based client that obtains and transmits three-dimensional user data, a makeup simulation unit that receives and stores the three-dimensional shape data from the shop-based client and carries out makeup simulation based on the three-dimensional shape data in response to requests from the user, and a server that includes a data control unit that analyzes the user's operation record and generates administrative information.

It is preferred that the shop-based client include a plurality of cameras by which to obtain images of the user from a plurality of viewpoints, a corresponding point search unit that receives each item of image data obtained from the plurality of cameras, analyzes the plurality of images, and searches for corresponding points that match each other, a three-dimensional shape recognition unit that analyzes the searched corresponding points and recognizes the three-dimensional shape of the object, a geometric calculation unit that sets a line of sight based on the recognition results from the three-dimensional shape recognition unit and generates an image from the prescribed line of sight through geometric conversion of the data based on the set line of sight, a display unit that displays the image generated by the geometric calculation unit, and a communication means that transmits the image data generated by the geometric calculation unit to the server.

It is further preferred that the makeup simulation unit of the server include a receiving unit that receives the three-dimensional shape data, a database that stores the received three-dimensional shape data, and a makeup simulation providing unit that provides a makeup simulation in response to requests for such simulation, and that the data control unit of the server include a user information analyzer that receives the operation history of the user and analyzes the trends therein, a control database that stores the analyzed data, an information processing unit that reads out data from the control database in response to external requests and processes the data in accordance with the requests, and a transmitting/receiving unit that transmits the output of the information processing unit to the requesting source and receives requests from the requesting source.

BRIEF DESCRIPTION OF THE DRAWINGS

Fig. 1 is a drawing showing the overall system pertaining to an embodiment of the present invention;

Fig. 2 is a drawing showing the basic construction of the server pertaining to the embodiment of the present invention;

Fig. 3 is a drawing showing the basic construction of the shop-based client pertaining to the embodiment of the present invention;

Fig. 4 is an example of the display screen of the shop-based client pertaining to the embodiment of the present invention;

Fig. 5 is a flow chart showing the basic outline of the processing performed by the image processing apparatus pertaining to the embodiment of the present invention;

Fig. 6 is a drawing to explain the operation principle cf the image processing apparatus pertaining to the embodiment of the present invention;

Fig. 7 is a drawing to explain the operation principle of the image processing apparatus pertaining to the embodiment of the present invention;

Fig. 8 is an external view of the image processing apparatus pertaining to the embodiment of the present invention;

Fig. 9 is a drawing to explain the operation principle of the image processing apparatus pertaining to the embodiment of the present invention;

Fig. 10 is a drawing to explain the operation principle of the image processing apparatus pertaining to the embodiment of the present invention;

Fig. 11 is a drawing to explain the operation principle of another image processing apparatus pertaining to the embodiment of the present invention;

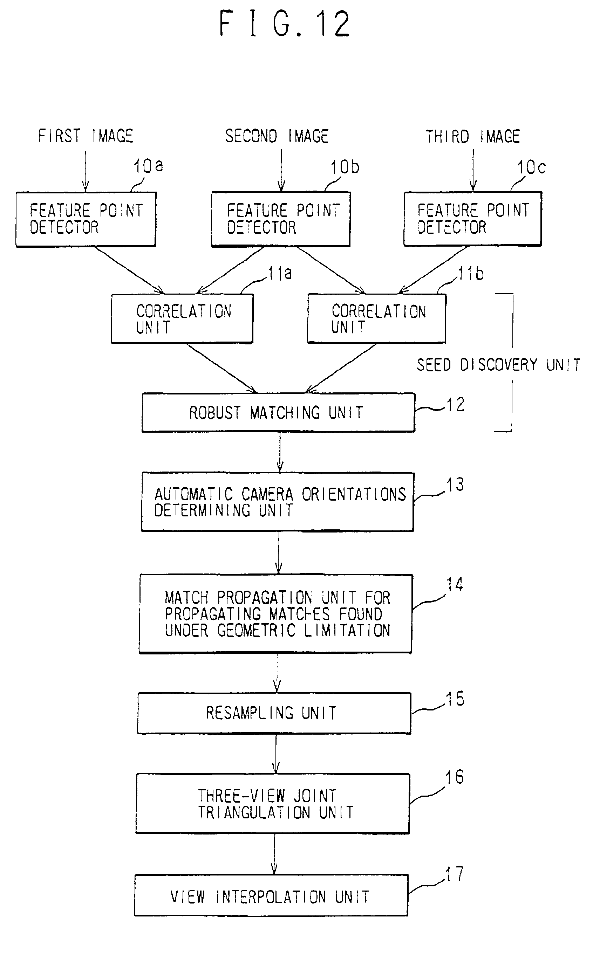

Fig. 12 is a summary block diagram of the image processing apparatus pertaining to the embodiment of the present invention;

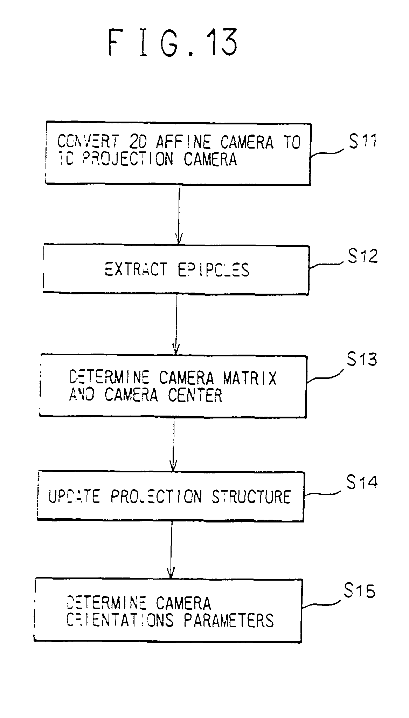

Fig. 13 is a flow chart showing the basic sequence to decide the camera orientations of the image processing apparatus pertaining to the embodiment of the present invention;

Fig. 14 is a flow chart showing an outline of the match propagation sequence carried out by the image processing apparatus pertaining to the embodiment of the present invention; and

Fig. 15 is a drawing to explain the principle of morphing.

## DESCRIPTION OF THE PREFERRED EMBODIMENTS

An embodiment of the present invention will be explained.

First, the concept of the present invention will be explained.

When a consumer accesses an Internet-based 'total beauty site' that is implemented by an embodiment of the present invention, data regarding the number of product click-throughs, etc. made by the consumer is obtained and analyzed, and a database is generated.

3D face model data (which includes not only the face but the overall body data) of the consumer is obtained by the apparatus located in the shop, and is stored in the server of the 'total beauty site'. A prescribed fee is paid by the shop to the operator of the site to cover such charges as a fee for use of the technology of the apparatus and the data, a franchise fee, the sales margin, and a consulting fee.

The site operator provides to manufacturers, magazine publishers and the like (1) consumer preference information derived from the number of click-throughs and (2) additional information classified by age, etc. Conversely, manufacturers, magazine publishers and the like pay the site operator consulting fees and data fees.

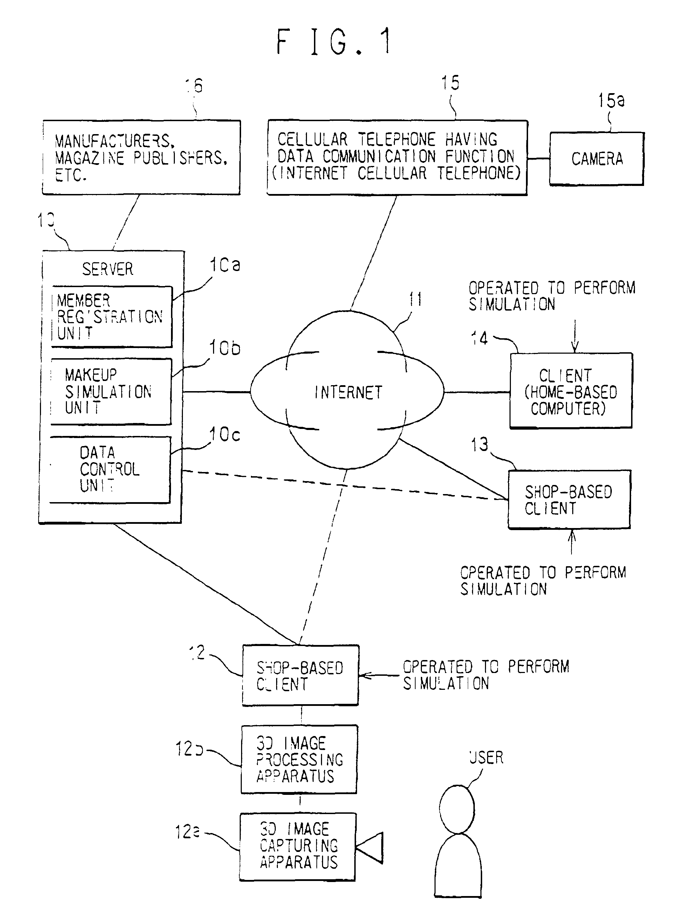

Next, a basic outline of the system pertaining to an embodiment of the present invention will be explained with reference to Figs. 1 through 4.

In Fig. 1, connected to the Internet 11 are a server 10 to operate the 'total beauty site', a shop-based client 12 to obtain 3D face model data, a shop-based client 13 to perform simulation, a client 14 by which a consumer can carry out a simulation at the consumer's home, and an Internet cellular telephone 15 having a camera 15a to obtain a 3D face model. A computer 16 belonging to a manufacturer, magazine publisher, etc. may be connected to the server 10. Furthermore, the shop-based clients 12 and 13 may be connected to the server 10 through a method other than the Internet 11.

In Fig. 1, the consumer obtains her own 3D face model data in a beauty shop using the 3D image capturing apparatus 12a and the 3D image processing apparatus 12b connected to the shop-based client 12. The specific procedure followed will be discussed below. The shop-based client 12 sends the obtained 3D face model data to the server 10. The server 10 stores the received data. Once the data is stored in the server, the consumer can access the 'total beauty site' on the server 10 and carry out a makeup simulation from the shop-based client 12, the shop-based client 13 that has no image capturing apparatus, the home-based personal computer 14 or the Internet cellular phone 15. The details of the makeup simulation will be described below. The behavior of the consumer while she is accessing the 'total beauty site' is analyzed by the data control unit 10c and accumulated in a database. Because this data comprises information important in understanding the consumer's preferences, it is provided by the server 10 to manufacturers, magazine publishers, etc. 16. Where the consumer has a camera-equipped computer or Internet cellular phone, a plurality of images obtained therefrom may sent to the server, enabling the server 10 to constructs 3D face model data. In the above explanation, the makeup simulation is carried out by the server 10. Because the simulation processing comprises advanced processing including three-dimensional processing, by having it carried out by a server 10 capable of advanced processing, the burden on the consumer-side personal computer may be reduced. It is acceptable if the makeup simulation is carried out by the shop-based client 12.

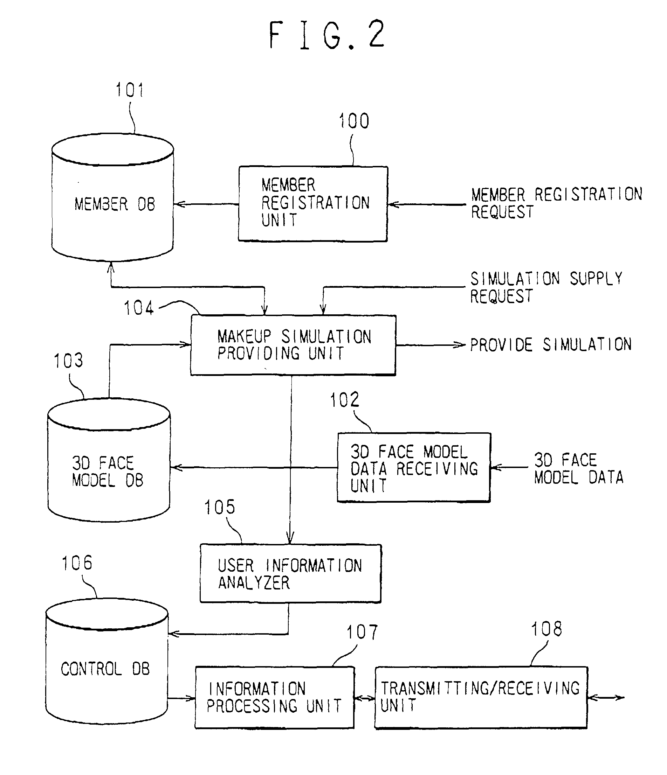

Fig. 2 shows the basic construction of the server 10. The member registration unit 10a shown in Fig. 1 includes the member registration unit 100 and member database 101 shown in Fig. 2. The makeup simulation unit 10b shown in Fig. 1 includes the 3D face model data receiving unit 102, the 3D face model database 103 and the makeup simulation providing unit 104 shown in Fig. 2. The data control unit 10c shown in Fig. 1 includes the user information analyzer 105, the control database 106, the information processing unit 107 and the transmitting/receiving unit 108 shown in Fig. 2.

When makeup simulation is carried out, member registration must first be performed. A member registration request is sent by the shop-based client 12 or the home-based computer 14 to the server 10. When the member registration request is received, the member registration unit 100 writes member information into the member database 101.

The 3D model face data sent by the shop-based client 12 is received by the receiving unit 102 and stored in the 3D face model database 103. When a simulation request is sent by the shop-based client 12 or 13 or the home-based computer 14 to the server 10, the makeup simulation providing unit 104 determines whether or not the request is from a member, and if the request is from a member, it analyzes the contents of the request, reads out the member data from the database 103, carries out simulation in accordance with the request, and provides the simulation to the requesting party.

At the same time, the actions taken by the consumer while at the 'total beauty site', for example, the contents of the simulation, clicks on specific products, clicks on banner ads, etc., are analyzed by the user information analyzer 105 and are organized and stored in the control database 106 as consumer preference information. When an information supply request is received by the transmitting/receiving unit 108 from a manufacturer, etc., the information processing unit 107 reads out prescribed data from the control database 106, subjects it to processing in accordance with the contents of the request, and then sends it to the requesting source. The details of the operation of the user information analyzer 105 and the information processing unit 107 are explained below.

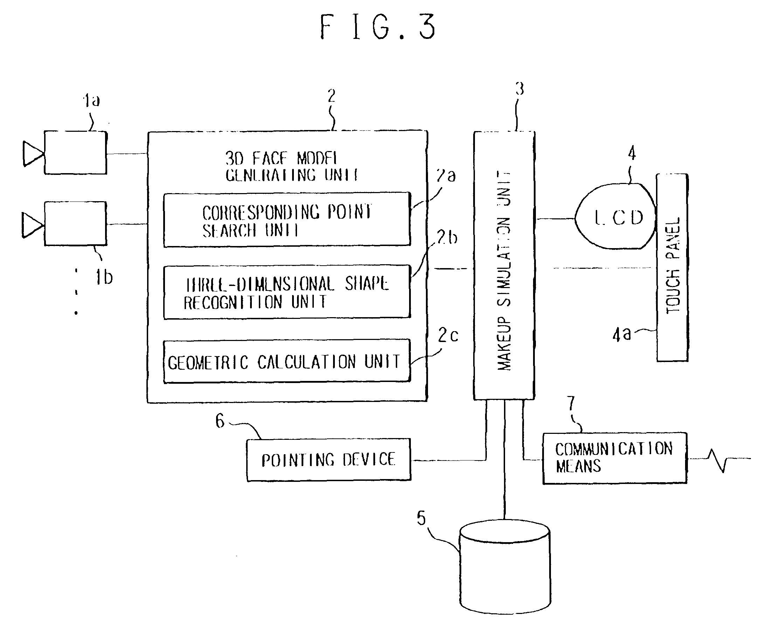

Fig. 3 shows the construction of the shop-based client 12. The shop-based client 12 includes a plurality of cameras 1a, 1b, etc., a 3D face model generating unit 2, a makeup simulation unit 3, a display unit 4, a touch panel 4a located on the display unit, a database 5 that stores 3D face model data, a pointing device 6 such as a mouse, and a communication means 7 that connects to the server 10 or the Internet 11. The 3D face model generating unit 2 includes a corresponding point search unit 2a, a three-dimensional shape recognition unit 2b and a geometric calculation unit 2c. Detailed actions of these units will be described later.

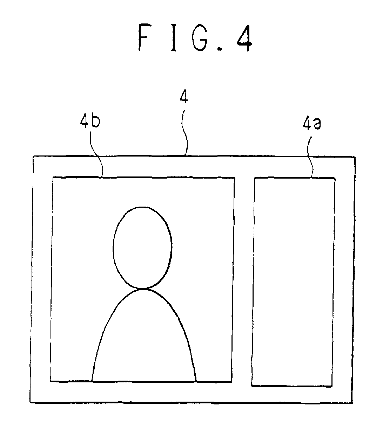

Fig. 4 shows the display apparatus 4. The three-dimensional image of the consumer is displayed in the area indicated by 4b, and color or pattern palette is shown in the touch panel 4a. Because a three-dimensional image is displayed in the display apparatus 4, a realistic makeup simulation may be experienced. For example, any type of makeup may be made through a one-touch operation of the touch panel 4a. A makeup style corresponding to various types of situations may be prepared in advance, such as party makeup, work makeup, etc., and the consumer's face may be reproduced with the new makeup style based on a single touch of the touch panel. Alternatively, a makeup simulation may be executed manually. Once 3D face model data is sent to the server 10, these simulations may be carried out on a personal computer at home.

Makeup simulation

The contents of a simulation carried out using the makeup simulation providing unit 104 and the makeup simulation unit 3 will now be explained.

Using this simulation, simulations of makeup, cosmetic surgery, clothing, perfume, accessories, hair style, etc., may be provided based on 3D information. In addition, using morphing technology as described below, information to enable one to resemble one's favorite model may be obtained. For example, intermediate images resembling a cross between oneself and one's favorite model may be created through morphing technology, and the desired image may be selected. The user can learn what percentage of the image comprises her own features and what percentage comprises the model's features. Simulation of not only one's face (the head area) but also one's entire body is possible as well.

Several specific examples of such a simulation will now be explained.

A simulation in which the level of beauty and degree of aging of the face are assessed and face identification is carried out will first be explained.

By analyzing the condition of ones facial skin and the light and dark areas reflecting the protrusions and indentations thereon, i.e., the state of the light areas and dark areas, the degree of one's beauty and apparent age can be objectively evaluated, identification of individual faces can be made, and the facial expression that best indicates and quantifies the person's emotional state can be objectively evaluated to a significant extent.

Each individual's face has its own particular light and dark areas and areas comprising a mixture thereof. This means that each individual's face may be recognized based on these light, dark and mixed areas. Furthermore, depending on how one observes the face, one can observe changes in the shape of a person's light and dark areas that occur with changes in a person's facial expression. In other words, one's facial expression changes with the contraction and relaxation of facial muscles, and such changes entail changes in the indentations and protrusions on one's face, i.e., in the light and dark areas of the face. Therefore, by observing these changes, even such an imprecise concept as a person's 'expression' can be quantified and objectively evaluated.

Accordingly, an evaluation facial image, which comprises a facial image that has undergone light/dark processing, is used. To create such an image, first, an image of the subject's face must be captured and a facial image obtained. Next, through image enhancement processing of this facial image, particularly image enhancement processing regarding the brightness of the image, an evaluation facial image comprising a plurality of areas having different levels of brightness is created. When this is done, the face may be evaluated for various purposes, such as the degree of beauty or of aging in the face, based on the contours of the light and dark areas in the evaluation image, or on the borders between these areas.

Furthermore, by comparing the evaluation facial image before and after face lift surgery, a determination of the degree of aging may be carried out.

Using the above processes, a simulation for plastic surgery or makeup styles may be performed.

Next, the process by which to correct the facial image will be explained. First, a desired face is chosen, images of a plurality of corrected candidate faces having varying degrees of resemblance to the desired face are created by alternating the original facial image using image processing such that the original facial image resembles the desired face image, and a corrected facial image is obtained by selecting from among these a plurality of corrected candidate facial images.

A model face may be used to choose the desired face. For the model face, a favorite television personality or actress may be used.

First, the desired face is selected. Where a makeup instructor is providing guidance regarding makeup application to a person wishing to wear makeup, for example, the desired face is chosen through the makeup instructor asking the prospective makeup wearer about her preferences. The desired face may be chosen using a model face. For the model face, the prospective makeup wearer can use the face of a favorite television personality or actress.

When the desired face is chosen, virtual makeup faces based on the desired face, i.e., images of virtual faces having the desired makeup style, are created. Through image processing such as the fusing of the desired face with the image of the face of the prospective makeup wearer, the faces of the prospective makeup wearer and of the desired face may be combined, bringing the prospective makeup wearer's face closer in appearance to that of the desired face. From these virtual makeup faces, the ideal makeup face that is most desired by the prospective makeup wearer is determined. Specifically, because images of a plurality of virtual makeup faces exhibiting varying degrees of fusion or resemblance between the prospective makeup wearer's face and the desired face are obtained through the above image processing, the preferred face can be chosen from among these faces as a desired virtual makeup face within the range of resemblance levels that may be obtained through the application of makeup. In this way, the ideal makeup face that is anticipated to be ultimately obtained may be provided beforehand. In other words, the prospective makeup wearer can learn the final made-up look in a short amount of time. By selecting the desired face forming the basis for the makeup and seeking the ideal makeup face to obtain in connection with this face in this way, the final made-up look may be displayed in a short amount of time.

Once the ideal makeup face is chosen through the above makeup simulation process, a makeup technique is deduced from the ideal makeup face. In other words, a series of makeup pointers by which to obtain the desired look, such as the areas where the eyebrows should be plucked or darkened, lines and areas where eye liner and eye shadow should be applied, eye shadow colors, areas where lipstick should be applied, and techniques for the application of foundation, are determined based on a preset makeup program. Makeup is then applied to the prospective makeup wearer's face based on these makeup pointers. As a result, the ideal makeup face, i.e., the look that was accepted beforehand by the prospective makeup wearer, may be accurately reproduced on the face of the prospective makeup wearer. Put another way, any makeup desired by the prospective makeup wearer can be applied on her face in a short period of time.

As described above, the makeup method of the present invention is characterized in that an ideal face based on a desired face, i.e., a model face, is created through image processing, and an important aspect of this method is that the current face of the prospective makeup wearer and the model face are incrementally combined and brought closer together through image processing.

Through this process, advanced corrections to the facial image may be made easily and in a short period of time. Furthermore, any desired makeup may be applied on the prospective makeup wearer in a short amount of time, and makeup possibilities based on a wide variety of cosmetic products may be effectively utilized.

Next, the simulation of makeup facial images in beauty parlors, cosmetics shops, beauty schools, etc. will be explained.

Makeup simulation drawing software uses a method in which the face as a whole is made up by applying makeup to individual parts of the face. In this method, the sought makeup style is pasted on. For example, regarding eyebrows, a method is used in which a given eyebrow shape is chosen and pasted onto the existing eyebrow after it is matched to the size of the eyebrow on the facial image. Similarly, where lipstick is applied to the lips, a method is used in which a pre-existing form is pasted on to an image.

For the model makeup, images of the eyebrows, lipstick on the lips, powder (including foundation, eye shadow and blush) on the skin, and colored contact lenses are drawn. The image drawing operation for each facial component is explained below.

- Eyebrows: The eyebrow area is defined, the eyebrow in the original eyebrow area is shaved off, and the color of the surrounding skin is drawn in. An eyebrow shape is chosen, and that shape is drawn in the eyebrow area. When this is done, processing is performed on a pixel-by-pixel basis in the eyebrow area, and the eyebrow is drawn in accordance with a defined calculation formula.

- Lipstick: The lip area onto which lipstick is to be applied is defined, and the chosen lipstick color is applied to the lip area. Here, image processing is carried out using the three elements of the hue, brightness and saturation. An image of the lip area is drawn by replacing the lip with the hue of the lipstick color, and converting the brightness and saturation of the original lips to the brightness and saturation of the lipstick. When this is done, operations such as glossing are also performed. Furthermore, the areas around the border between the lips and the skin are drawn such that the border between the lips and skin is a continuous color.

- Powdering of the skin: An image is drawn in which the skin color value and the powder color value are mixed according to a specified ratio. Here, powdering includes the application of makeup such as foundation, eye shadow and blush.

- Colored contact lenses: After the positions at which colored contact lenses are to be placed in the image (the positions at which the colored parts of the contacts are to be drawn) are defined, the color values of the colored contact lenses and of the iris are mixed according to a defined ratio in the display.

The following variables should be recorded as makeup information for the model on whom makeup is applied using the above methods:

- Eyebrows - eyebrow shape, eyebrow color (color value), position and size relative to the face

- Lipstick - lipstick color value, degree of gloss

- Powder - powder color value and density of application on a pixel-by-pixel basis

- Colored contact lenses - color value of colored contact lenses

- Facial image definition points

The processing to apply the makeup of the selected model to the facial image of the user is carried out according to the following procedure. First, in a preliminary step, the facial image of the user is loaded into a computer using a digital camera, etc., and the facial image is defined to match the image set for the model. Afterward, the same attribute values used for the model's makeup are loaded in and applied to the defined facial image of the user. While the makeup is different for each facial component, the same materials are used for the user's eyebrows, lipstick and colored contact lenses that were used for the model, and images are drawn in the user's facial image using the same methods that were used for the drawing of the model face. However, regarding powdering, because the density and the type of powder may differ depending on the area of the face, the correspondences of the respective pixels of the facial images of the model and the user are obtained, and makeup is applied to the facial image of the user. Specifically, using morphing technology, the correspondence of the respective pixels of the model facial image and the user's facial image is calculated, and the same powder that was present in a given pixel of the model image is used for the pixel of the user's skin having the same skin attribute at the same pixel of the model image.

When a desired model facial image is selected from among the plurality of model facial images displayed on the screen (where the makeup varies even though the same model is used, the different images are displayed as a separate menu item), the eyebrows, lipstick, and colored contact lenses are automatically applied using the methods explained with regard to the model makeup. However, because the powder application can differ on a pixel-by-pixel basis, powder is applied using morphing technology. Two techniques are used in morphing; warp and dissolve. The first involves a method by which, when changing from one shape to another, the corresponding points are sought and the original shape is transformed, while the second involves transformation of the image by mixing the pre-change color and the post-change color in accordance with a defined ratio. The image drawing carried out for powdering in the present invention uses warp.

It is often difficult to determine precisely what type of makeup one desires. Therefore, it is useful to have a model makeup style to refer to. When several models are registered beforehand, the makeup style used by the model can be applied to the user's facial image simply through the selection of a particular model, and therefore makeup styles that the user likes, or model makeup styles that might be applied to one's face, can be selected, and many different makeup styles may be 'tried on'.

Not only can the makeup style that was applied to the model facial image be transferred to the user's facial image, but because makeup is applied while preserving the user's facial contours and skin features, different impressions may be created even with the same makeup style. For example, powder applied to a pale-skinned model reflects light differently than powder applied to a user's suntanned skin. Consequently, the best makeup style for one's own face can be sought through simulation on a screen.

Once the user's facial image data is loaded into a computer via an image data input apparatus and the facial area definition is made, simulation may be performed by changing the model an unlimited number of times. Furthermore, the skin color, the condition of the lips, or the quality of the face itself can change between a facial image taken in summer and a facial image taken in winter. In the present invention, because makeup is applied while the characteristics of the original facial image are preserved, and these characteristics may change from time to time as described above, the effect of the makeup at different times may be checked and confirmed even if the same makeup is applied (i.e., the same model is selected) to the face of the same person. For example, because the method of the present invention by which lipstick is drawn on the lips preserves the lines and shading of the lips even after the application of lipstick, one can clearly see the difference in the effect of the lipstick between when it is applied on rough lips during winter and when it is applied on fresh lips during summer. In the case of skin in particular, because skin color differs in summer and winter, the effect of makeup varies depending on the type of powder used. Using the automatic makeup simulation of the present invention, the differences in the effect of makeup based on the condition of the user's face may be directly confirmed on a screen as described above. Therefore, if facial images taken in the four different seasons are used, the best makeup style may be found in a short amount of time by applying the makeup styles of various different models on one's facial image for the current season.

User information analyzer

Next, the user information analyzer 105 and the user processing unit 107 will be explained. These units carry out the following processes:

(1) Statistical compilation of the user's Web usage information (number of click-throughs), etc.)
(2) Analysis of the Web usage information, analysis of not just purchasing information but preference information
(3) Aggregation of purchasing information, supply of product preference information in new form
(4) Analysis cross-referenced by age and region information

The user information analyzer 105 extracts, organizes and supplies data by which to understand the overall user information based on the contents of the member database 101. It performs classification of all of the registered users, and outputs basic user characteristic data such as the total number of registered users, the ratio of men to women, the distribution of users by age and location of residence, etc. From the user behavior history, which includes information on the degree of cooperation of each user with questionnaire surveys and on the frequency with which the user purchased products through the Internet home page, the class of users that would be best selected as target users may be learned.

If the attributes of the target user class are clearly established, an effective business may be developed by matching with the preferences of the target user class such basic elements as the contents of the 'total beauty site', the style of writing, and the merchandise offered. Furthermore, problems may become more clearly defined. An example of such a problem might be that although women were originally targeted, there are fewer female registered users than expected. In such a case, responsive actions such as the posting of banner ads in information portals or sites accessed by a large number of women may be taken.

It is also possible to prepare a number of different brochures that are custom-tailored to the attributes of each group of users, and to send brochures with different contents to each group. One such brochure might focus on information regarding the most popular products among the merchandise handled by the 'total beauty site'. A better response would be anticipated in such a case that would be expected when the same brochure with the same content is sent to all members on a global basis.

The user information analyzer 105 performs access analysis. 'Access analysis' is the most basic analysis that measures the number of people that visit a particular site. If a site is equated to a shop, this number is equivalent to the number of customers visiting the shop. Analysis from various viewpoints may be carried out. For example, trends may be obtained regarding the number of customers visiting on each day of the week or during each time period of the day, the number of customers who enter but leave without purchasing, and the number of customers visiting each area of the site.

Access analysis is performed using the three indices of number of hits, PV (page view), and number of visitors.

The number of hits is a value that indicates the number of 'data sets' that were requested to be sent from a particular site. The unit of measurement for 'data sets' here is the number of data files in a computer. If the data set is a home page and the home page includes a large amount of graphic data, the number of hits increases accordingly. Conversely, even if a large amount of information is contained in one page, if that data consists of one text file, it is counted as '1' hit.

A more practical index is PV (page view). It indicates the total number of Internet home pages viewed in connection with a particular site. While this index entails the shortcoming that any single home page counts as 1 PV regardless of the amount of information contained therein, it is a standard index used to measure the value of a medium or the effect of an ad, such as a banner ad, that is displayed on a one-page basis.

There are cases in which the number of PVs associated with the top page of a particular site is deemed the number of visitors. Because PV indicates the number of total viewed pages, the number of different people that have viewed the page cannot be obtained. The number of visitors is an index for solving this problem. Naturally, where one person accesses the top page repeatedly, each access is counted, and therefore, the number of visitors in this case is only an approximate number.

In order to measure the number of visitors more precisely, such methods as a 'cookie' or a 'registration system' must be used.

A cookie not only enables behavior analysis, but is also effective for one-to-one marketing. The use of a cookie allows the behavior of a particular person (or more accurately, the behavior of a Web browser) within the site to be tracked.

For example, suppose it is learned that consumers who request a lipstick simulation during a makeup simulation session are significantly more likely to request lipstick brochures than consumers who do not request a lipstick simulation.

If this trend is utilized properly, the target population may be approached more effectively. If a brochure request page is forcibly shown to users who request a lipstick simulation, the rate of brochure requests may be increased substantially.

Through the use of a cookie, information may be provided in a customized fashion that matches each user's behavior and preferences. In order to implement this feature, the site must have cookie issuance and database functions.

While personalization based on the use of a cookie cannot completely specify each individual, a registration system can overcome this shortcoming.

The address, telephone number, e-mail address and name are registered beforehand, and an ID and password used exclusively by the 'total beauty site' are issued. A member accessing a site enters a member-only page when she inputs her ID and password.

By having the users log in, the identity of each user, the pages they visit, and their behavior while logged in can be tracked by the site. At the same time, a page dedicated to the user may be displayed after login.

If the areas of information desired by a user are obtained through responses to a questionnaire distributed at the time of registration, news that matches the user's stated interests may be posted on a particular page.

From not only the registration information, but also from behavior information that indicates the areas of the site most commonly visited by the user, the individual's preferences may be derived and information matching these preferences may be displayed.

Three-dimensional face model generating unit

The three-dimensional face model generating unit 2 will now be explained.

One known method of image processing is the morphing technique. Morphing is a computer graphics (CG) technology developed in Hollywood, U.S.A. According to this method, two different images are used, for example, images of the faces of two persons, and one of the images is gradually changed on the screen to the other image, thereby providing a series of images showing such change. Using the morphing technology, it is possible to create a series of images in which, for example, a white tiger turns into a young woman.

When two images A and B are given, the morphing process is roughly as follows. First, the corresponding feature points between image A and image B are obtained (e.g., eye and eye, nose and nose). This process is normally performed by an operator. When the correspondences are found, feature point p of image A is gradually changed in a time-consuming process to feature point q of image B, resulting in the image series as described above,

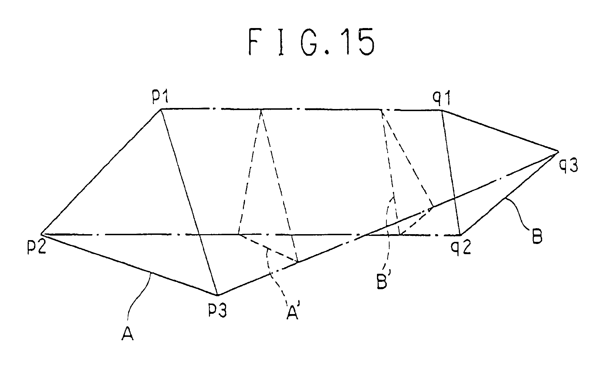

In CG, an image is generally made of a large number of triangular elements. Therefore, morphing is performed by changing the triangle of feature point p in image A to the triangle of feature point q in image B while maintaining the correspondence between them. This will be described further with reference to Fig. 15. In this figure, triangle A is part of image A, and triangle B is part of image B. The apexes p1, p2, p3 of triangle A each correspond to apexes q1, q2 and q3 of triangle B. In order to convert triangle A to triangle B, the differences between p1 and q1, p2 and q2, and p3 and q3 are calculated, and then respectively added to each of the apexes p1, p2, p3 of triangle A. By adding all (100%) of these differences, triangle A is converted to triangle B. It is also possible to add portions of these differences instead of the whole differences, e.g., 30% or 60% thereof. In such case, the intermediate figures between triangle A and triangle B can be obtained. For example, in Fig. 15, triangle A' is a model example of an addition of 30% of the difference, and triangle B' is a model example of an addition of 60% of the difference.

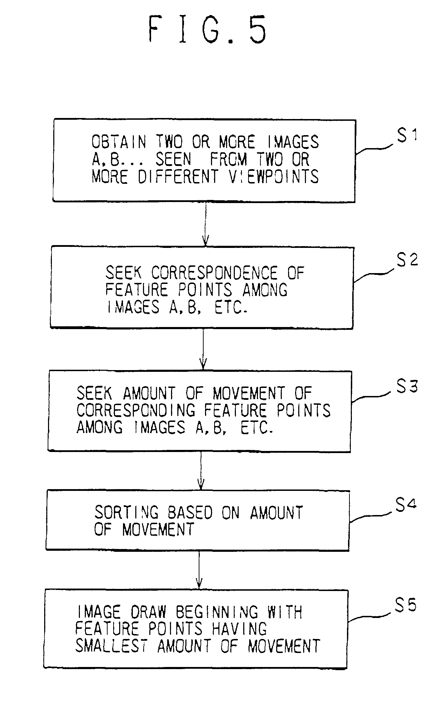

Fig. 5 is a flowchart showing an outline of the processing of the apparatus/method according to an embodiment of the present invention. The image data (signals) obtained from a plurality of cameras 1a, 1b, in Fig.3. are input into a front view image generating unit 2. In front view image generating unit 2, a corresponding point searching unit 2a searches the mutually corresponding points by analyzing the plurality of images. These corresponding points are analyzed by a three-dimensional shape identifying unit 2b, and the three-dimensional shape of the object is identified. Based on the identified results, the viewing rays are set, and the data is geometrically converted or varied based on the set viewing rays, thereby generating a front view image that would be gained by looking into a mirror. Each of the above-mentioned processes will be described in further detail below. Furthermore, camera 1 need only be a plurality of cameras, regardless of whether 2, 3, 4 or more. Two or three are desirable from the practical aspect.

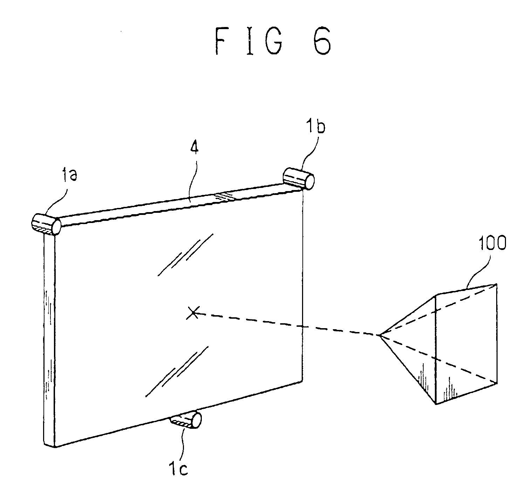

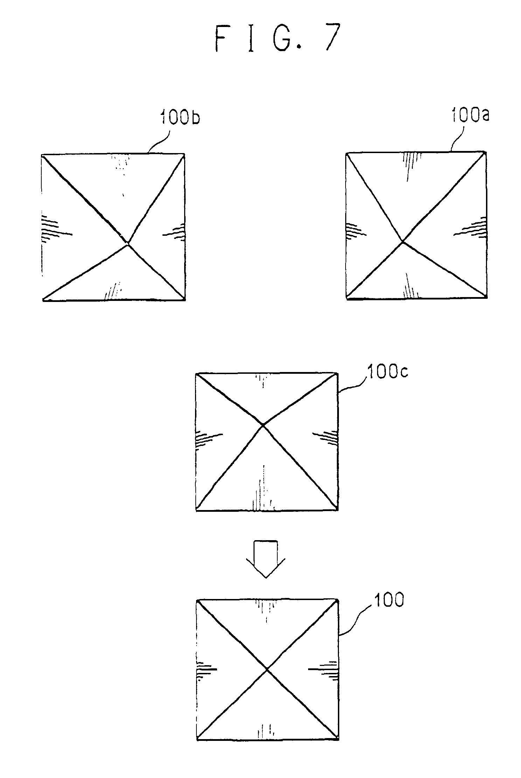

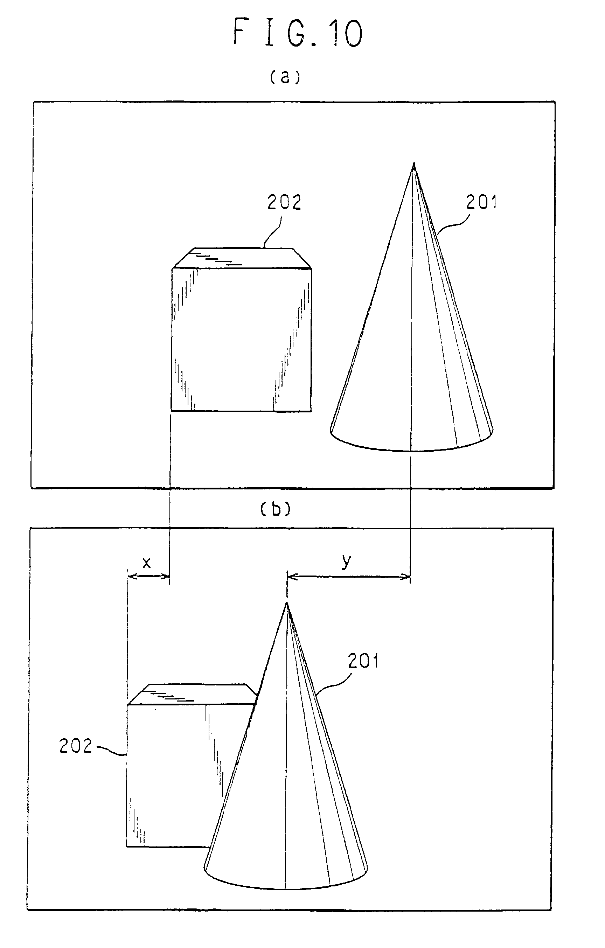

The processing of the front view image generating unit will be described in further detail based on Figs. 6 and 10. Fig. 6 is a model view of a digital mirror comprising cameras 1 at the left and right upper ends and the lower center of a plate-shaped liquid crystal display apparatus (LCD) 4. An object 100 is placed on the normal vector intersecting substantially the center of LCD 4. Normally, the face of the user is located at this position, but for convenience of explanation, a quadrangular pyramid is used as an example. When quadrangular pyramid 100 is shot by cameras 1a, 1b, and 1c, images 100a, 100b, and 100c are obtained. Image 100a is shot by camera 1a, and viewed from LCD 4, this image is a view of pyramid 100 from the left side. Image 100b is shot by camera 1b, and is a view of pyramid 100 from the right side. Image 100c is shot by camera 1c, and is a view of pyramid 100 from the bottom. If there are at least two images seen from different viewpoints located relatively adjacent to each other, then it is possible to identify a unique three-dimensional shape from a plurality of two-dimensional images through a geometrical calculation processing similar to the stereoscopic view processing. In order to perform this processing by a computer, it is necessary to specify the feature points. In the present example, the apexes of quadrangular pyramid 100 are selected. When the feature points have been specified for all images, the correspondence between these feature points is calculated. In this way, it is analyzed at which position in each image the same portion of pyramid 100 is located. Based on this analysis, the three-dimensional shape of pyramid 100 is identified. According to image 100a, the apex is on the left side, so it is clear that pyramid 100 is at the left of cameral 1a. In this way, the three-dimensional shape is identified. Thereafter, the viewpoint is set for example substantially in the center of LCD 4, and based on this viewpoint, an image of pyramid 100 is generated. For example, image 100 as shown in Fig. 7 is obtained.

In Fig. 3, signal processing unit 3 receives the front view image processed as above from front view image generating unit 2, and performs various processing such as displaying the object or a reflection of the object such as gained by conventional mirror reflection, etc. Examples are the zoom and wide angle processes. A certain portion of the whole image reflected in a mirror is instantaneously enlarged or reduced. The selection of the portion to be enlarged or reduced and the processing to be performed is designated by a pointing device 6 such as a mouse. If the surface of LCD 4 is a touch panel, it is possible to touch an arbitrary portion of the image to enlarge or reduce such portion instantaneously.

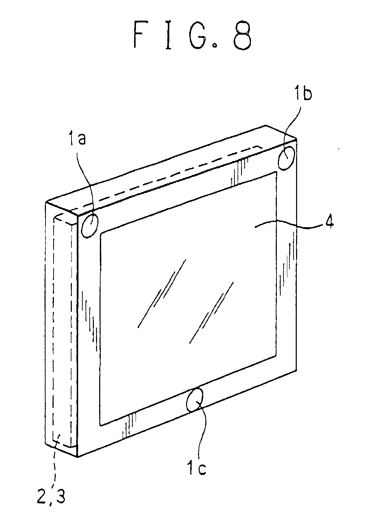

Fig. 8 is a variation of the apparatus in Fig. 3. Three CCD cameras 1a, 1b and 1c are provided around LCD 4. At the back of LCD 4, a computer is provided which functions as front view image generating unit 2 and signal processing unit 3. These are all stored in one case.

Now, the whole processing of the apparatus/method according to an embodiment of the present invention will be described in outline. According to the flowchart in Fig. 5, two or more images A, B,... from two or more different viewpoints are obtained (S1).

Next, the correspondence between feature points in image A and image B is calculated (S2). Feature points may be edges, corners, texture, etc.

The difference between corresponding feature points in image A and image B is calculated (S3). Through this processing, the extraction of the necessary features points and the difference between them (amount of change) can be gained as required for the morphing process.

The present embodiment is also a drawing apparatus and method for performing morphing of images of three-dimensional objects. In order to draw images of three-dimensional objects, the position of the object within a space must be determined, and, according to the present drawing apparatus and method, it is possible to draw images of three-dimensional objects without directly requiring the three-dimensional position.

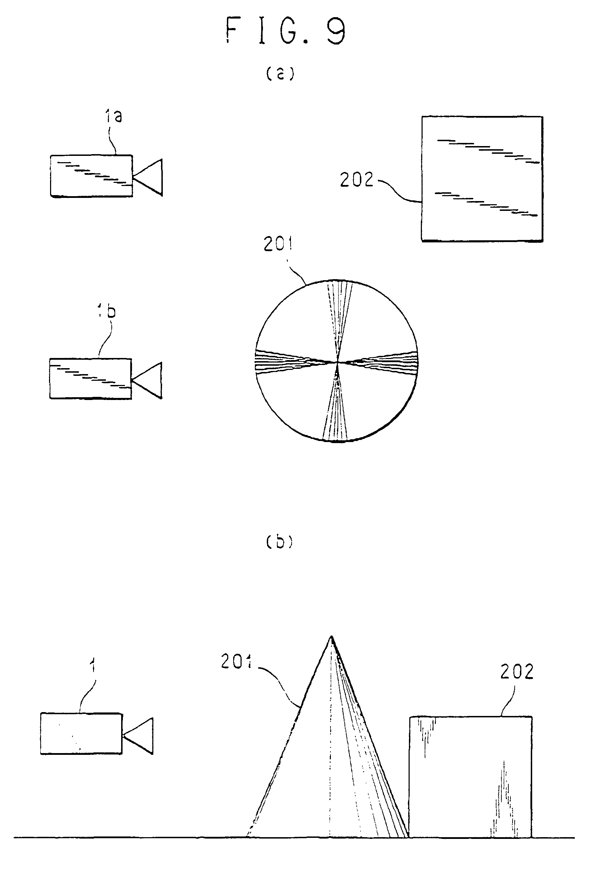

The movement principle will be described by using Figs. 9 and 10. As shown in Figs. 9(a) and (b), a cone 201 and a cube 202 are arranged within a certain space and shot by two cameras 1a and 1b. As the viewpoints of cameras 1a, 1b differ, the obtained images are also different. The images obtained by cameras 1a, 1b are as shown in Figs. 10(a) and (b). Comparing these two images, it is clear that the positions of cone 201 and cube 202 are different. Assuming that the amount of change in the relative position of cone 201 is y, and that of cube 202 is x, then Fig. 10 shows that x<y. This is due to the distance between the object and the cameras. If the values of x and y are large, the feature points are near the camera. On the other hand, if such values are small, the feature points are far from the camera. In this way, the distances between the object and the cameras are clear from the differences between corresponding feature points in the different images. Utilizing this characteristic, the feature points are sorted according to the differences (S4), and the images are written in order from that with the smallest difference (meaning the image shot by the camera farthest to the object) to the largest difference (S5). Portions near the camera are overwritten and displayed, but portions far from the camera (hidden portions) are deleted through the overwriting. In this way, it is possible to adequately reproduce an image in three-dimensional space without using depth information.

The apparatus shown in Figs. 5 to 8 is able to display an image shown from a different viewpoint than camera 1 by processing the image obtained from camera 1 as shown in Fig. 5. For example, it is possible to use the images of a face from the right, from the left and the bottom to generate and display the image of the face seen from the front. Also, by applying morphing processing to the face seen from the right and the left, it is possible to display the face from various angles, as if the camera viewpoint had continuously moved. The apparatus in Figs. 5 to 8 can be used quasi as a digital form mirror (hereinafter the "digital mirror").

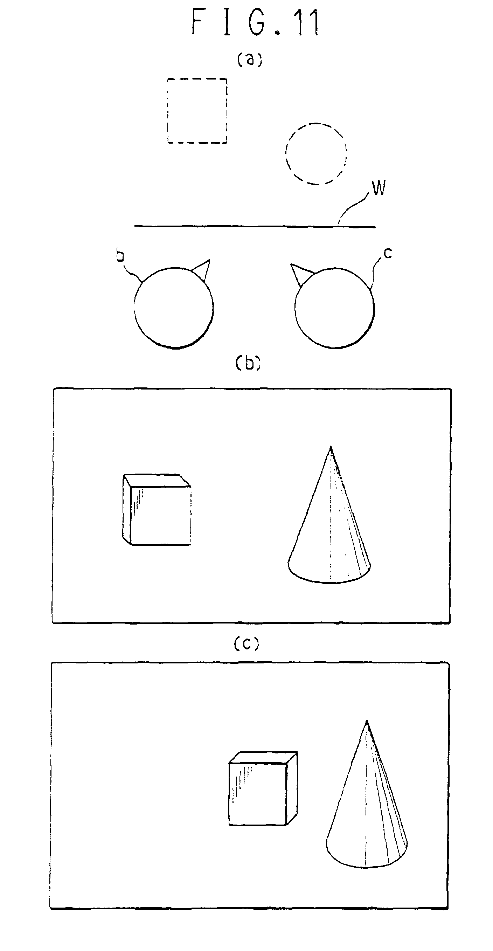

It is also possible to use the apparatus in Figs. 3 to 5 as a digital window simulating an actual window. By displaying various scenes on a liquid crystal television, the present invention provides a display apparatus for the window to be used in substitution of the actual window. Conventional display apparatuses merely displayed images, e.g. scenery, seen from a fixed viewpoint, being unable to express small changes in scenery occurring from changes in the viewpoint position at the actual window. By utilizing the apparatus or method according to the present embodiment, it is possible to recognize the position of the person, i.e. the position of the viewpoint, so by changing the display according to the viewpoint position, an even more real scenery display is possible. For example, Fig. 11 shows a liquid crystal apparatus ("digital window") W and a person standing before it. In Fig. 11(a), a cone and cube are arranged within a virtual space, and this situation is displayed on liquid crystal apparatus W. If the person is at position b, the image shown in Fig. 11(b) will be displayed on liquid crystal apparatus w, and if the person is at position c, then the image shown in Fig. 11(c) will be displayed. In this way, by displaying an adequate screen according to the viewpoint position, the user will feel as if he were turning his head at an actual window.

The digital mirror and digital window processing methods above are common in that they include a processing of determining the position of an object within a three-dimensional space by calculating the correspondence of feature points between a plurality of images. In the digital mirror, the position measurement precision is desirably high, as the measurement precision of the three-dimensional position directly affects the image precision. However, in the digital window, there is no large feeling of strangeness even if the viewpoint position is somewhat inaccurate. Therefore, the digital window does not require as high a measurement precision of the position as the digital mirror. A processing apparatus/method for the digital mirror will be hereinafter referred to as the facial image generator and a processing apparatus/method for the digital window the scenery image generator. Both will be now described in further detail.

The facial image generator conducts its processing using three cameras and a trifocal tensor suited as constraint. The scenery generator conducts its processing using two cameras and the epipolar geometry as constraint. Conventionally, it was difficult to find correspondences only by comparing the three images of the three cameras, but by using the space constraints of the three cameras, the correspondence search can be performed automatically.

**Facial Image Generator**

An example of the processing of three images with different viewpoints from three cameras will be described below.

1. Feature point detection unit

Three images with different viewpoints are input into three feature point detection units 10a to 10c. Feature point detection units 10a to 10c outputs a list of feature points also called points of interest. If the object has a geometrical shape such as triangles or squares, the apexes thereof are the features points. In normal photograph images, points of interest are naturally good candidates for feature points as points of interest are by their very definition image points that have the highest textureness.

2 Seed finding unit

Correlation units lla and llb and a robust matching unit 12 make a seed finding unit. This unit functions to find an aggregate of initial trinocular matches (constraint of the positions of three cameras) that are highly reliable. Three lists of points of interest are input into this unit, and the unit outputs a list of trinocular matches of the points of interest called seed matches. Correlation units lla and 11b establish a list of tentative trinocular matches. Robust matching unit finalizes a list of reliable seed matches using robust methods applied to three view geometric constraints.

2.1 Correlation unit
The movements of correlation units lla and 11b will be described below. These units perform the processing of three lists of points of interest in three images output from feature point detection unit 10a to 10c. The ZNCC (zero-mean normalized cross-correlation) correlation measure is used for finding correspondences. By using the ZNCC correlation measure, it is possible to find the correspondence between images even if the size of the object is somewhat different between such images or the images are somewhat deformed. Therefore, the ZNCC correlation is used for matching seeds.

The $ZNCC_x(\Delta)$ at point $x = (x,y)^T$ with the shift $\Delta = (\Delta_X, \Delta_Y)^T$ is defined to be:

$$\frac{\Sigma_i(I(x + i) - \bar{I}(x))(I'(x + \Delta + i) - \bar{I}'(x + \Delta))}{(\Sigma_i(I(x + i) - \bar{I}(x))^2 \, \Sigma_i(I'(x + \Delta + i) - \bar{I}'(x + \Delta))^2)^{1/2}}$$

where $\bar{I}(x)$ and $\bar{I}'I(x)$ are the means of pixel luminances for the given window centered at x.

2.2 Robust matching unit
Next, the binocular matches from correlation unit 11 are merged into one single trinocular match by robust matching unit 12. Robust matching unit 12 receives input of a list of potential trinocular matches from correlation unit 11 and outputs a list of highly reliable seed trinocular matches. A robust statistics method based on random sampling of 4 trinocular matches in three images is used to estimate the 12 components of the three-view constraints to remove the outliers of trinocular matches. When the same object is shot by three cameras and three images from different viewpoints are gained, the same point in the object in each of the three images (e.g., position of feature point) can be uniquely defined from the position of the object, the camera position and the camera direction according to certain rules. Therefore, by determining whether the points of interest in the list of trinocular matches gained from correlation unit 11 satisfies such rules, it is possible to obtain the list of points of interest of the correct trinocular matches.

Given $u = (u,v)$, $u' = (u',v')$ and $u'' = (u'',v'')$ the normalized relative coordinated of the trinocular matches, the three-view constraints are completely determined by the following 12 components $t_1$ to $t_{12}$:

$$t_4\ u + t_8\ v + t_{11}\ u' + t_9\ u'' = 0,$$

$$t_2\ u + t_6\ v + t_{11}v' + t_{10}u'' = 0,$$

$$t_3\ u + t_7\ v + t_{12}u' + t_9\ v'' = 0,$$

$$t_1\ u + t_5\ v + t_{12}v' + t_{10}v'' = 0.$$

3 Unit of auto-determination of camera orientations

Now, a camera orientation auto-determination unit 13 will be described below. The classical off-line calibration of the whole system is hardly applicable here even though 3 cameras may be a priori fixed, but their orientations could be still variable. Therefore, camera orientation auto-determination unit 13 determines the camera orientation in order to constrain the match propagation. In other words, camera orientation auto-determination unit 13 receives input of a list of seed matches from robust matching unit 12 and outputs the orientation of the camera system.

Now, the basic ideas of camera orientation auto-determination unit 13 will be described below. At first, the three-view constraints $t_1,...,t_{12}$ are optimally re-computed by using all trinocular inlier matches. The extraction of camera orientations directly from the three-view constraints for later usage is based on an original observation that the problem of affine cameras is converted into a nice problem of 1D projective cameras.

For those skilled in the art, it is evident that an elegant 1D projective camera model first introduced in L. Quan and T. Kanade "Affine structure from line correspondences with uncalibrated affine cameras" IEEE Transactions on Pattern Analysis and Machine Intelligence, 19(8): 834-845, August 1997 occurs on the plane at infinity for the usual affine cameras. All directional quantities are embedded on the plane at infinity, therefore encoded by the 1D projective camera. The 1D camera is entirely governed by its trifocal tensor $T_{ijk}$ (providing a strong constraint) such that $T_{ijk}u^iu'^ju''^k = 0$.

From the above aspects, the procedure of determining the camera orientations according to the present embodiment is as follows.

S11: Convert 2D affine cameras into 1D projective cameras
Using tensor-vector mapping defined by $4(a - 1) + 2(b - 1) + c$ 1 between the tensor components and the three-view constraint components converts the triplet of affine cameras represented by ti into the triplet of 1D cameras represented by $T_{abc}$.
S12: Extraction of epipoles
The 1D camera epipoles can be extracted from the tensor by solving, for instance, $|T_{.jk}e_2| = 0$ for the epipoles $e_2$ and $e_3$ in the first image. The other epipoles can be similarly obtained by factorizing the matrix $T_{i.k}e'_1$ for $e'_1$ and $e'_3$ and $T_{.jk}e''_1$ for $e''_1$ and $e''_2$.
S13: Determination of camera matrices $M' = (H, h)$ and $M'' = (H', h')$ and the camera centers c, c' and c''
It is first straightforward that $h = e'_1$ and $h' = e''_1$. The homographic parts of the camera matrices are determined from $T_{ijk} = H_i^jh^k - h'^jH'_i^k$. Then, the camera centers and the 2D projective reconstruction can be determined from the camera matrices as their kernels.
S14: Update of the projective structure
The known aspect ratio for the affine camera is equivalent to the knowledge of the circular points on the affine image plane. The dual of the absolute conic on the plane at infinity could be determined by observing that the viewing rays of the circular points of each affine image plane are tangent to the absolute conic through the camera center.
S15: Determination of camera orientation parameters
Transforming the absolute conic to its canonical position therefore converts all projective quantities into their true Euclidean counterparts. Euclidean camera centers give the orientation of the affine cameras and the affine epipolar geometry is deduced from the epipoles.

4. Constraint match propagation unit

Now, a constraint match propagation unit 14 for expecting a maximum number of matches in three images will be described below. This unit 14 receives input of a list of seed matches and camera orientation parameters from camera orientation auto-determination unit 13 and outputs dense matching in three images.

After obtaining the initial seed matches, it comes the central idea of match propagation from the initial seed matches. The idea is similar to the classic region growing method for image segmentation based on the pixel homogeneity. The present embodiment adopts region growing to match growing. Instead of using the homogeneity property, a similarity measure based on the correlation score is used. This propagation strategy could also be justified as the seed matches are the points of interest that are the local maxima of the textureness, so the matches could be extended to its neighbors which have still strong textureness though not a local maxima.

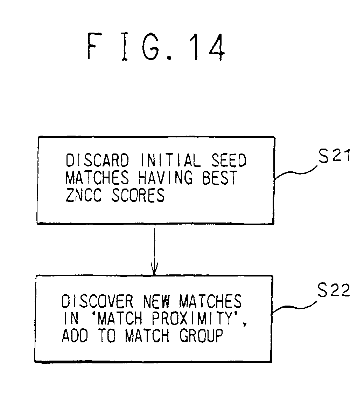

All initial seed matches are starting points of concurrent propagations. At each step, a match (a, A) with the best ZNCC score is removed from the current set of seed matches (S21 in Fig. 14). Then new matches are searched in its 'match neighborhood' and all new matches are simultaneously added to the current set of seeds and to the set of accepted matches-under construction (S22). The neighbors pixels a and A are taken to be all pixels within the 5 x window centered at a and A to ensure the continuity constraint of the matching results. For each neighboring pixel in the first image, we construct a list of tentative match candidates consisting of all pixels of a 3 x 3 window in the neighborhood of its corresponding location in the second image. Thus the displacement gradient limit should not exceed 1 pixel. This propagation procedure is carried out simultaneously from the first to the second and the first to the third image, and the propagation is constrained by the camera orientation between each pair of images. Only those that satisfy the geometric constraints of the camera system are propagated. Further, these two concurrent propagations are constrained by the three-view geometry of the camera system. Only those that satisfy the three-view geometry of the camera system are retained.

The unicity constraint of the matching and the termination of the process are guaranteed by choosing only new matches not yet accepted. Since the search space is reduced for each pixel, small 5 x 5 windows are used for ZNCC, therefore minor geometric changes are allowed.

It can be noticed that the risk of bad propagation is greatly diminished by the best first strategy over all matched seed points. Although seed selection step seems very similar to many existing methods for matching points of interest using correlation, the crucial difference is that propagation needs only to take the most reliable ones rather than taking a maximum of them. This makes our algorithm much less vulnerable to the presence of bad seeds in the initial matches. In some extreme cases, only one good match of points of interest is sufficient to provoke an avalanche of the whole textured images.

5. Re-sampling unit

Now, a re-sampling unit 15 will be described below. The dense matching may still be corrupted and irregular, re-sampling unit 15 will regularize the matching map and also provide a more efficient representation of images for further processing. Re-sampling unit 15 receives input of the dense matching in three images from constraint match propagation unit 14 and outputs a list of re-sampled trinocular matches.

The first image is initially subdivided into square patches by a regular grid of two different scales 8 x 8 and 16 x 16. For each square patch, we obtain all matched points of the square from the dense matching. A plane homography H is tentatively fitted to these matched points $u_i \longleftrightarrow u'_i$ of the square to look for potential planar patches. A homography in $P^2$ is a projective transformation between projective planes, it is represented by a homogeneous 3 x 3 non singular matrix such that $\lambda_i u'_i = Hu_i$, where u and u' are represented in homogeneous coordinates. Because a textured patch is rarely a perfect planar facet except for manufactured objects, the putative homography for a patch cannot be estimated by standard least squares estimators. Robust methods have to be adopted, which provide a reliable estimate of the homography even if some of the matched points of the square patch are not actually lying on the common plane on which the majority lies. If the consensus for the homography reaches 75%, the square patch is considered as planar. The delimitation of the corresponding planar patch in the second and the third image is defined by mapping the four corners of the square patch in the first image with the estimated homography H. Thus, a corresponding planar patches in three images is obtained.

This process of fitting the square patch to a homography is first repeated for all square patches of the first image from the larger to the smaller scale, it turns out all matched planar patches at the end.

6 Three-view joint triangulation unit

Now, a three-view joint triangulation unit 16 will be described below. The image interpolation relies exclusively on image content without any depth information and is sensitive to visibility changes and occlusions. The three view joint triangulation is designed essentially for handling the visibility issue. Three-view joint triangulation unit 16 receives input of the re-sampled trinocular matches and outputs joint three-view triangulation. The triangulation in each image will be Delaunay because of its minimal roughness properties. The Delaunay triangulation will be necessarily constrained as we want to separate the matched regions from the unmatched ones. The boundaries of the connected components of the matched planar patches of the image must appear in all images, and therefore are the constraints for each Delaunay triangulation.

The joint three-view triangulation is defined as fulfilling the following conditions.

- There is one-to-one vertex correspondence in three images.
- The constraint edges are the boundary edge of the connected components of the matched regions in three images.
- There is one-to-one constraint edge correspondence in three images.
- In each image, the triangulation is a constraint Delaunay triangulation by the constraint edges.

A natural choice to implement this joint three-view triangulation is a greedy-type algorithm.

7 View interpolation unit

Now, a view interpolation unit 17 will be described below. According to view interpolation unit 17, any number of in-between new images can be generated, for example, images seen from positions between a first and a second camera. These in-between images can be generated from the original three images. View interpolation unit 17 receives input of the three-view joint triangulation results and outputs any in-between image $I(\alpha, \beta, \gamma)$ parameterized by $\alpha$, $\beta$, and $\gamma$ such that $\alpha+\beta+\gamma=1$.

The view interpolation processing is performed according to the following procedures.

1. The position of the resulting triangle is first interpolated from three images.
2. Each individual triangle is warped into the new position and a distortion weight is also assigned to the warped triangle.
3. Each whole image is warped from its triangulation. In the absence of depth information, a warping order for each triangle is deduced from its maximum disparity to expect that any pixels that map to the same location in the generated image are arriving in back to front order as in the Paiter's method. All unmatched triangles are assigned the smallest disparity so that they are always warped before any matched triangles.
4. The final pixel color is obtained by bleeding three weighted warped images.

Furthermore, the similar idea developed for facial image generation from 3 images could be extended to either 2 or N images with reasonable modification of the processing units. Other objects than face images could also be processed in a very similar manner.

Needless to say, the present invention is not limited to the embodiment described above and may be varied within the scope of the invention described in the claims, and such variations are included within the scope of the present invention.

As used herein, means is not limited to physical means but includes cases where the functions of such means are realized through software. Furthermore, the functions of one means may be realized through two or more physical means, and the functions of two or more means may be realized through one physical means.

**Claims**

1. A three-dimensional beauty simulation client-server system comprising:

   a shop-based client that obtains and transmits three-dimensional shape data regarding a user; and
   a server that comprises a makeup simulation unit that receives and stores said three-dimensional shape data from said shop-based client and carries out makeup simulation based on said three-dimensional shape data in response to the user's requests, and a data control unit that analyzes the user's operation record and generates administrative information.

2. The three-dimensional beauty simulation client-server system according to claim 1, further comprising a client that can access said server, wherein said server provides a makeup simulation in response to requests from said client.

3. The three-dimensional beauty simulation client-server system according to claim 1, further comprising a cellular telephone that has a data transmission function and can access said server, wherein said server provides a makeup simulation in response to requests from said cellular telephone.

4. The three-dimensional beauty simulation client-server system according to claim 1, wherein said server further comprises a member registration unit that stores member registration information, and wherein said server provides makeup simulations to users registered beforehand in said member registration unit.

5. The three-dimensional beauty simulation client-server system according to claim 1, wherein said server transmits the operation record and/or administrative information regarding said users via a computer network.

6. The three-dimensional beauty simulation client-server system according to claim 1, wherein said shop-based client comprises:

a plurality of cameras to obtain images of the user as seen from a plurality of viewpoints;

a corresponding point search unit that receives each item of image data obtained from the plurality of cameras, analyzes the plurality of images, and searches for corresponding points that correspond to each other;

a three-dimensional shape recognition unit that analyzes the searched corresponding points and recognizes the three-dimensional shape of the target object;

a geometric calculation unit that sets a line of sight based on the recognition results from said three-dimensional shape recognition unit, and generates an image from a prescribed line of sight through geometric conversion of the data based on the set line of sight;

a display unit that displays the image generated by said geometric calculation unit; and

communication means that transmits the image data generated by said geometric calculation unit to said server.

7. The three-dimensional beauty simulation client-server system according to claim 6, wherein said corresponding point search unit and said geometric calculation unit comprises:

a feature point extraction unit that extracts feature points from each of said plurality of images;

a correlation calculating unit that seeks correlation among the feature points of said plurality of images and seeks combinations of said feature points;

a matching unit that discards combinations having a low feasibility from among said combinations of feature points based on the condition that the images were seen from said plurality of viewpoints;

a camera orientations determining unit that seeks the positions of said plurality of viewpoints and the directions of the lines of sight; and

a match propagation unit that, under the conditions imposed by the positions of said plurality of viewpoints and the direction of said lines of sight obtained by said camera orientations determining unit, selects combinations of feature points starting with those having superior geometric and statistical reliability and adjusts the analysis range of the images of said target object.

8. The three-dimensional beauty simulation client-server system according to claim 6, wherein said corresponding point search unit and said geometric calculation unit comprises:

a feature point extraction unit that extracts feature points from each of said plurality of images;

a correlation calculating unit that seeks correlation among the feature points of said plurality of images and seeks combinations of said feature points;

a matching unit that discards combinations having a low feasibility from among said combinations of feature points based on the condition that the images were seen from said plurality of viewpoints;

a camera orientations determining unit that seeks the positions of said plurality of viewpoints and the directions of the lines of sight;

a match propagation unit that, under the conditions imposed by the positions of said plurality of viewpoints and the direction of said lines of sight obtained by said camera orientations determining unit, selects combinations of feature points starting with those having superior geometric and statistical reliability and adjusts the analysis range of the images of said target object;

a resampling unit that normalizes the matching map obtained by said match propagation unit;

a three-dimensional position measurement unit that determines the position of said target object in a three-dimensional space based on the normalized matching map; and

a view interpolation unit that generates images seen from viewpoints different from said plurality of viewpoints based on the determined three-dimensional position of said target object.

9. The three-dimensional beauty simulation client-server system according to claim 6, wherein said corresponding point search unit and said geometric calculation unit comprises:

a feature point extraction unit that extracts feature points from each of said plurality of images;

a correlation calculating unit that seeks correlation among the feature points of said plurality of images and seeks combinations of said feature points;

a matching unit that discards combinations having a low feasibility from among said combinations of feature points based on the condition that the images were seen from said plurality of viewpoints; and

a match propagation unit that, under the geometric constraints imposed by the lines of sight, selects combinations of feature points starting with those having superior geometric and statistical reliability and adjusts the analysis range of the images of said target object.

**10.** The three-dimensional beauty simulation client-server system according to claim 6, wherein said corresponding point search unit and said geometric calculation unit comprises:

a feature point extraction unit that extracts feature points from each of said plurality of images; a correlation calculating unit that seeks correlation among the feature points of said plurality of images and seeks combinations of said feature points;

a matching unit that discards combinations having a low feasibility from among said combinations of feature points based on the condition that the images were seen from said plurality of viewpoints;

a match propagation unit that, under the geometric constraints imposed by the lines of sight, selects combinations of feature points starting with those having superior geometric and statistical reliability and adjusts the analysis range of the images of said target object;

a resampling unit that normalizes the matching map obtained by said match propagation unit; a three-dimensional position measurement unit that determines the position of said target object in a three-dimensional space based on the normalized matching map; and

a view interpolation unit that generates images seen from viewpoints different from said plurality of viewpoints based on the determined three-dimensional position of said target object.

**11.** A three-dimensional beauty simulation server comprising a makeup simulation unit that receives and stores three-dimensional shape data of a user from a shop-based client and carries out makeup simulation based on the three-dimensional shape data in response to requests from the user, and a data control unit that analyzes the operation record for said user and generates administrative information,

wherein said makeup simulation unit comprises a receiving unit that receives said three-dimensional shape data, a database that stores the received three-dimensional shape data, and a makeup simulation providing unit that provides a makeup simulation in response to requests for such simulation; and wherein said data control unit of said server comprises a user information analyzer that receives the operation history of the user and analyzes the trends therein, a control database that stores the analyzed data, an information processing unit that reads out data from the control database in response to external requests and processes the data in accordance with said requests, and a transmitting/receiving unit that transmits the output of said information processing unit to the requesting source and receives requests from the requesting source.

**12.** The three-dimensional beauty simulation server according to claim 11, wherein said makeup simulation providing unit analyzes the condition of the user's facial skin and the light and dark areas that indicate the protrusions and indentations thereon, and evaluates the user's facial expression based on the results of such analysis.

**13.** The three-dimensional beauty simulation server according to claim 11, wherein said makeup simulation providing unit obtains a facial image of the user, displays for the user a plurality of target facial images stored beforehand for allowing the user to select one of these images, combines said user facial image and said target facial image in a plurality of predetermined ratios, and supplies a plurality of combined facial images to the user.

**14.** The three-dimensional beauty simulation server according to claim 11, wherein said makeup simulation providing unit supplies facial images seen from freely chosen viewpoints.

**15.** A system comprising means for deriving a 3-D image of a user, means storing information regarding user preferences regarding certain predetermined criteria, and means for generating an image of the user modified in accordance with said stored information.

**16.** A method of producing a modified image of a user, the method comprising determining information relating to user preferences regarding certain predetermined criteria, deriving a 3-D image of a user, and modifying the image of the user in accordance with said information.

**17.** A method as claimed in claim 16 wherein the user's behaviour is monitored to determine said information relating to user preferences.

EP 1 134 701 A2

# F I G . 1

MANUFACTURERS, MAGAZINE PUBLISHERS, ETC.

CELLULAR TELEPHONE HAVING DATA COMMUNICATION FUNCTION (INTERNET CELLULAR TELEPHONE)

CAMERA

SERVER

MEMBER REGISTRATION UNIT

MAKEUP SIMULATION UNIT

DATA CONTROL UNIT

INTERNET

OPERATED TO PERFORM SIMULATION

CLIENT (HOME-BASED COMPUTER)

SHOP-BASED CLIENT

OPERATED TO PERFORM SIMULATION

SHOP-BASED CLIENT

OPERATED TO PERFORM SIMULATION

3D IMAGE PROCESSING APPARATUS

USER

3D IMAGE CAPTURING APPARATUS

EP 1 134 701 A2

# FIG.2

# F I G. 3

3D FACE MODEL
GENERATING UNIT

CORRESPONDING POINT
SEARCH UNIT

THREE-DIMENSIONAL SHAPE
RECOGNITION UNIT

GEOMETRIC CALCULATION UNIT

MAKEUP SIMULATION UNIT

LCD

TOUCH PANEL

POINTING DEVICE

COMMUNICATION
MEANS

EP 1 134 701 A2

# F I G . 4

# F I G . 5

OBTAIN TWO OR MORE IMAGES A,B... SEEN FROM TWO OR MORE DIFFERENT VIEWPOINTS — S1

SEEK CORRESPONDENCE OF FEATURE POINTS AMONG IMAGES A,B, ETC. — S2

SEEK AMOUNT OF MOVEMENT OF CORRESPONDING FEATURE POINTS AMONG IMAGES A,B, ETC. — S3

SORTING BASED ON AMOUNT OF MOVEMENT — S4

IMAGE DRAW BEGINNING WITH FEATURE POINTS HAVING SMALLEST AMOUNT OF MOVEMENT — S5

# F I G 6

# F I G. 7

FIG. 8

1a

1b

4

1c

2,3

# FIG. 9

(a)

(b)

# F I G. 10

(a)

(b)

# F I G. 11

(a)

W

b

c

(b)

(c)

# F I G. 12

FIRST IMAGE  SECOND IMAGE  THIRD IMAGE

```
FEATURE POINT    FEATURE POINT    FEATURE POINT
DETECTOR  10a    DETECTOR  10b    DETECTOR  10c
```

```
CORRELATION  11a    CORRELATION  11b
UNIT                 UNIT
```

SEED DISCOVERY UNIT

ROBUST MATCHING UNIT  12

AUTOMATIC CAMERA ORIENTATIONS DETERMINING UNIT  13

MATCH PROPAGATION UNIT FOR PROPAGATING MATCHES FOUND UNDER GEOMETRIC LIMITATION  14

RESAMPLING UNIT  15

THREE-VIEW JOINT TRIANGULATION UNIT  16

VIEW INTERPOLATION UNIT  17

# F I G . 13

```
┌─────────────────────────────┐
│ CONVERT 2D AFFINE CAMERA TO  │──── S11
│ 1D PROJECTION CAMERA         │
└─────────────────────────────┘
            │
            ▼
┌─────────────────────────────┐
│      EXTRACT EPIPOLES        │──── S12
└─────────────────────────────┘
            │
            ▼
┌─────────────────────────────┐
│  DETERMINE CAMERA MATRIX     │──── S13
│  AND CAMERA CENTER           │
└─────────────────────────────┘
            │
            ▼
┌─────────────────────────────┐
│  UPDATE PROJECTION STRUCTURE │──── S14
└─────────────────────────────┘
            │
            ▼
┌─────────────────────────────┐
│  DETERMINE CAMERA            │──── S15
│  ORIENTATIONS PARAMETERS     │
└─────────────────────────────┘
```

# F I G. 14

DISCARD INITIAL SEED MATCHES HAVING BEST ZNCC SCORES — S21

DISCOVER NEW MATCHES IN 'MATCH PROXIMITY', ADD TO MATCH GROUP — S22

F I G.15